# EUROPEAN PATENT APPLICATION

(11) **EP 4 834 876 A1**
(43) Date of publication of application: **04.11.2026**
(21) Application number: 25382434.6
(22) Date of filing: 29.04.2025
(51) Int. Cl.: C07C 227/18, C07C 231/10, C07C 231/12, C07C 253/30, C07C 257/06, C07C 257/14, C07D 233/70, C07D 241/08, C07C 229/16, C07C 237/22, C07C 237/12, C07C 255/29, C07C 255/25

(54) **A GLYCONITRILE-BASED CHEMICAL REACTION NETWORK**

(71) Applicant: Universidad Autónoma de Madrid, 28049 Madrid (ES); Consejo Superior de Investigaciones Científicas (CSIC), 28006 Madrid (ES)
(72) Inventor: DE LA ESCOSURA NAVAZO, Andrés, 28049 Madrid (ES); VELA GALLEGO, Sonia, 28049 Madrid (ES); VALDÉS TABERNERO, Alberto, 28049 Madrid (ES); CIFUENTES GALLEGO, Alejandro, 28049 Madrid (ES)
(74) Representative: Hoffmann Eitle

(57) **Abstract**

The present invention refers to a glyconitrile chemical reaction network, its implementation and its use in the search for new biologically active substances.

## Description

### TECHNICAL FIELD OF THE INVENTION

The present invention refers to a glyconitrile-based chemical reaction network, a method of implementing such a chemical reaction network, a method for modulating such a chemical reaction network, and the use of such a chemical reaction network for the preparation and investigation of new biologically active molecules and their potential reaction pathways.

### BACKGROUND

The preparation of compounds with interesting biological or pharmacological properties has been based traditionally on complex multistep synthetic routes, normally directed towards an individual target molecule. The design of these syntheses relies on retrosynthetic analyses, in which the target molecule is recursively broken down into increasingly simple precursors to find possible pathways. However, retrosynthesis is a complicated task and requires a lot of experience and excellent chemical skills to obtain good results later when applied in practice.

Once a possible pathway has been identified, it must be proven by synthesising the target product first in the laboratory, and, if successful, then at larger scale. This presents sustainability problems, both in academic and industrial R&D labs, because its development requires a lot of trial and error, which is time, resources and energy consuming. The scale up of the lab synthesis then produces even larger amounts of waste during the development phase, since the reaction might behave different at gram scale versus kilogram scale. For example, 25-100 kg of waste per kg of product for pharmaceutical drugs is typical. This means that for the standard synthesis of new drug candidates a lot of resources and material is required, without having the certainty that this drug candidate will indeed have the expected biological activity and safety and/or will comply with all regulatory requirements expected from a new drug.

Combinatorial chemistry was suggested as a way to prepare a high number of potential molecules in a more efficient and automated manner by combining different building blocks. This represents a planned parallel synthesis approach that is carried out in separate reaction vessels. However, although this approach allows the synthesis of many molecules at the same time, it requires a specialised equipment and infrastructure for the automated synthesis, and it does not allow for studying new possible synthetic pathways.

Hence, there is a need for new strategies in chemistry that allow for the preparation, identification and testing of new biologically active molecules in a more efficient way. At the same time, there is also a need to identify all potential synthetic pathways towards the new biologically active molecules in order to find the most promising ones and the most robust ones for a later industrial development that can be based upon experimental evidence and not only on theoretical retrosynthetic analysis. It would be highly desired to perform these activities in one single process. The present inventors have now surprisingly discovered that chemical reaction networks (CRN) offer an opportunity in this respect.

Most of the reaction networks studied so far involve systems with a biological origin, including metabolic networks, protein folding networks, artificial genetic networks, signal transduction pathways, microbial consortia, biogeochemical cycles, enzymatic cascade processes and DNA- or peptide-based synthetic networks. In the chemical field on the other hand, only very few CRNs have been described, such as oscillating reactions, atmospheric chemistry networks and reaction-diffusion systems. None of them has however a clear synthetic potential, and the discovery and handling of networks comprising organic compounds with the aim to identify and make new pharmaceutical drugs remain still a huge challenge due to the lack of understanding how the interplay between individual reactions and the overall network organization determines the complex behaviour of chemical systems. Yet, chemical systems offer a unique opportunity, based on the bottom-up construction of chemical networks from simple reactive molecular building blocks.

In recent years, the introduction of automation, microfluidics, integrated analytics, and artificial intelligence-based integration of big data has led to programmable syntheses of molecules enabled by computer-controlled devices. The help of Big Data and artificial intelligence (AI) allows understanding the complexity and hyperdimensionality of chemical and biological systems. Within the umbrella of big data, the different omics techniques, and in particular metabolomics, are key in the investigation of biological systems with complex architectures at the molecular level. In many cases, this implies the use of machine learning and other Al tools. Because these tools are suitable for addressing complex problems involving massive combinatorial spaces and non-linear behaviours, they are also ideal for addressing one of today's major scientific and technological challenges, the long-term purpose of implementing life-like features in chemical reaction networks (CRNs). Being able to study massive data generated in a controlled and recursive way from CRNs could represent a new powerful methodology towards artificial living systems and materials and help understanding how specific molecules may be prepared and obtained reliably and in good yield. This opens now new opportunities for chemical synthesis of multiple compounds using CRNs, instead of synthesising individual compounds.

One of the very few studied CRNs with synthetic potential is based on the formose reaction. This formose CRN represents a process that allows obtaining dozens of sugars from formaldehyde with calcium hydroxide as catalyst. The group of Wilhelm Huck recently showed in their article "Environmental conditions drive self-organization of reaction pathways in a prebiotic reaction network" in Nature Chemistry, Vol. 14, June 2022, p. 623-631 by William E. Robinson, Elena Daines, Peer van Duppen, Thijs de Jong and Wilhelm T. S. Huck how different environmental inputs can drive the formose system towards self-organized and structured compositions, in which the different reaction pathways interact dynamically. This behaviour is consistent with the emergence of subsets of molecules within the network that respond coherently to variables such as catalyst availability, temperature and concentrations of the raw materials. However, the formose CRN is not very well suited for pharmaceutical lead research, because it is aimed only at sugar molecules and lacks the structural variability necessary for screening possible drug candidates.

Hence, there is still a lack of CRNs based on organic compounds. In particular, there is a need for CRNs that are generated from simple raw materials but offer a high degree of tuneability to provide rapidly enough information about new potential biologically active ingredients and metabolites and their possible synthetic pathways.

### SUMMARY OF THE INVENTION

Here a glyconitrile chemical reaction network and its implementation is proposed that overcomes the limitations of the prior art and enables a fast and simultaneous exploration and optimization of multiple reaction pathways towards biologically active organic compounds occurring in the chemical reaction network and based on experimental evidence.

In a first aspect, a method for implementing a glyconitrile chemical reaction network is provided, the glyconitrile chemical reaction network being generated from a set of starting compounds comprising (a) glyconitrile; (b) a salt selected from the group consisting of alkali halides, alkaline earth metal halides, or mixtures thereof; and (c) a base selected from the group consisting of alkali hydroxides, alkaline earth metal hydroxides, ammonia, monoalkyl amines, dialkyl amines, trialkyl amines, alkali phosphates, alkaline earth metal phosphates, alkali carbonates, alkaline earth metal carbonates, or mixtures thereof.

In a second aspect, a glyconitrile chemical reaction network for providing multiple compounds is provided, wherein the glyconitrile chemical reaction network is generated from a set of starting compounds comprising (a) glyconitrile; (b) a salt; and (c) a base, wherein the initial (a) glyconitrile concentration is from 250 mM to 1 M, the initial (b) salt concentration is from 100 mM to 1 M, and the initial (c) base concentration is from 30 mM to 750 mM.

In a third aspect, a method for continuously modulating a glyconitrile chemical reaction network is provided comprising the steps of providing at least one chemical input by means for a continuous reaction system having at least one inlet channel, analysing the composition of the glyconitrile chemical reaction network by means for analysis, and varying one or more chemical reaction conditions selected from the group of temperature, agitation speed, pH, reagent concentration, or reaction time.

In a fourth aspect, a process is provided for preparing amino acids, nucleobases, nucleotides, oligopeptides, and/or metabolite derivatives thereof using a glyconitrile chemical reaction network, comprising the steps of generating and/or implementing a glyconitrile CRN, analysing the CRN composition after a defined set time, identifying a desired amino acid, nucleobase, nucleotide, oligopeptide, and/or metabolite derivative thereof, and modulating the reaction conditions in a way to predominately produce the desired amino acid, nucleobase, nucleotide, oligopeptide, and/or metabolite derivative thereof.

In a fifth aspect, a use of the glyconitrile chemical reaction network of the previous aspects is provided for a multi-compound screening of biologically active substances.

In a sixth aspect, a device configured for implementing a glyconitrile chemical reaction network of the previous aspects is provided comprising (i) means for the preparation of the glyconitrile chemical reaction network, (ii) means for sampling the network composition one or more times at defined time points providing one or more sample datasets, (iii) means for analysing the one or more sample datasets, including means for data acquisition and data extraction, providing one or more analysis datasets, (iv) means for data processing of the obtained one or more analysis datasets, and (v) means for compound identification.

Preferred aspects may be derived from the following description, examples and claims.

### DRAWINGS

**Figure 1** illustrates the central position of glyconitrile with respect to key prebiotic synthetic routes and the accessibility to different types of compounds.

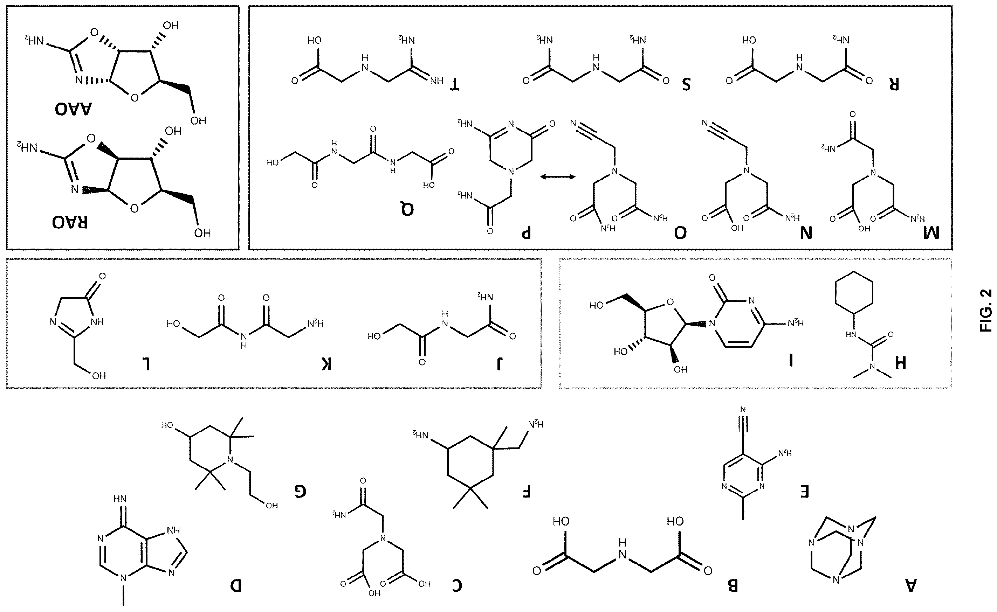

**Figure 2** illustrates exemplary compounds that were identified in the glyconitrile CRN: Compounds (A) hexamethylenetetramine, (B) iminodiacetic acid, (C) N-(2-acetamido)iminodiacetic acid, (D) 3-methyladenine, (E) 4-amino-2-methylpyrimidine-5-carbonitrile, (F) isophoronediamine and (G) 1-(2-hydroxyethyl)-2,2,6,6-tetramethyl-4-piperidinol were confirmed with authentic standards; compounds (H) 3-cyclohexyl-1,1-dimethylurea and (I) cytarabine were tentatively identified based on MS/MS spectral database or retention time (RT) library match; compounds (J) 2-[(2-hydroxyacetyl)amino]acetamide, (K) 2-amino-N-(2-hydroxyacetyl)acetamide and (L) 2-(hydroxymethyl)-1,4-dihydroimidazol-5-one were tentatively identified by using different bioinformatics tools; compounds (M) [Bis(2-amino-2-oxoethyl)amino]acetic acid, (N) 2-[(2-amino-2-oxoethyl)-(cyanomethyl)amino]acetic acid, (O) 2-[(2-amino-2-oxoethyl)-(cyanomethyl)amino]acetamide, (P) 1(2H)-pyrazineacetamide,5-amino-3,6-dihydro-3-oxo-, (Q) 2-[[2-[(2-hydroxyacetyl)amino]acetyl]amino]acetic acid, (R) 2-((2-amino-2-oxoethyl)amino)acetic acid, (S) 2-[(carbamoylmethyl)amino]acetamide and (T) 2-[(2-amino-2-iminoethyl)amino]acetic acid were tentatively identified based on the manual interpretation of MS/MS spectra; ribose aminoxazoline (RAO) and arabinose aminoxazoline (AAO) were identified when additional chemical inputs were added to the reaction.

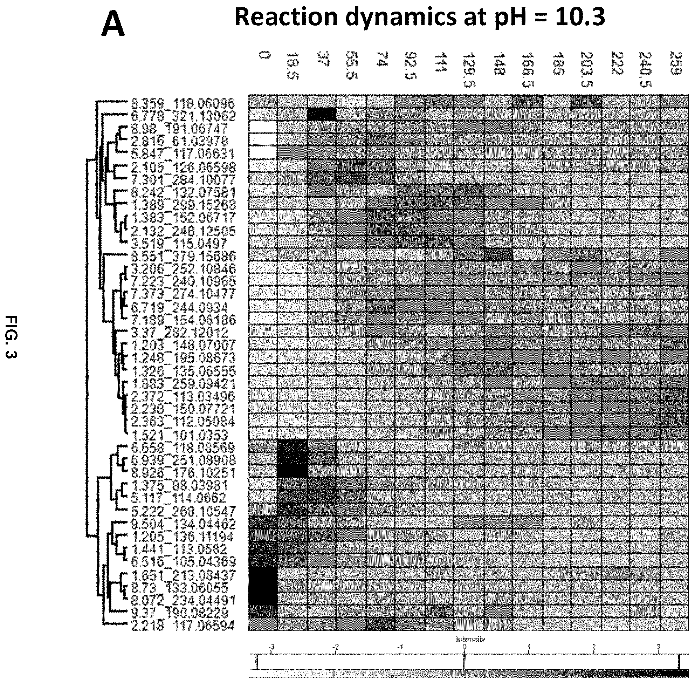

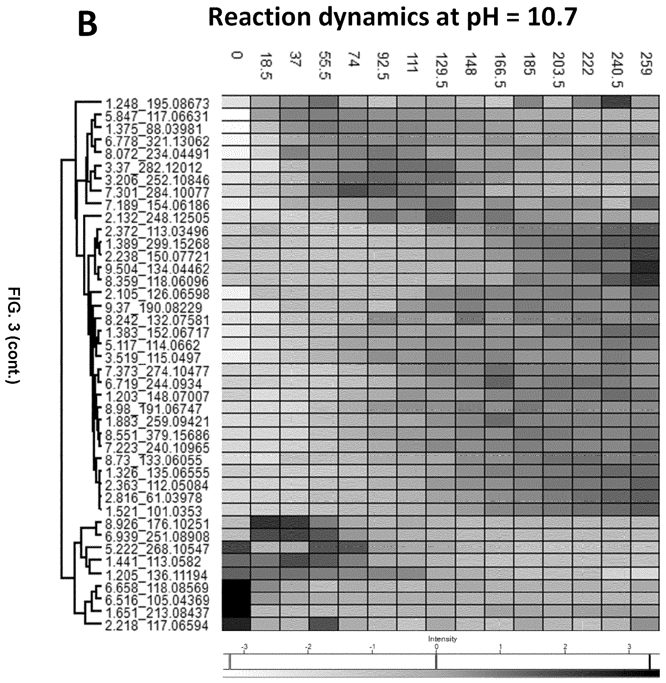

Figure 3 illustrates in A) a heatmap for the reaction dynamics of the glyconitrile CRN initially set at pH = 10.3 and in B) a heatmap for the reaction dynamics of the glyconitrile CRN initially at pH = 10.7. These heatmaps show the distribution of relative RT_m/z intensities (after Z-score normalization) for a list of 42 species selected from the glyconitrile CRN at different times. Each column represents a fingerprint of the output composition that may be used for further applications. The compounds are clustered based on their reaction dynamics during the experiment. The scalebars of relative intensities are depicted at the bottom of the graphs and the colour code used is white for lowest intensity and black for highest intensity.
**Figure 4** illustrates a matrix of chemical input conditions designed to evaluate the influence of different variables on the glyconitrile CRN composition in batch.
**Figure 5** illustrates PCA score plots for samples prepared containing different concentrations of glyconitrile (0.52, 0.42 and 0.32 M) and NaOH (0.11, 0.075 and 0.05 M), incubated at different temperatures (40, 55 and 70 °C), and grouped according to (**A**) temperature-glyconitrile concentration pairs, or (**B**) temperature-NaOH concentration pairs.
**Figure 6** is a dendrogram representation of the signals obtained from the samples prepared containing different concentrations of glyconitrile (0.52, 0.42 and 0.32 M) and NaOH (0.11, 0.075 and 0.05 M), and incubated at different temperatures (40, 55 and 70 °C) in batch mode. Pie plots placed on the 'leaf' positions represent the normalized average product distributions of the 13 compounds depicted on the right panel. Longer paths between leaves represent lower similarity.
**Figure 7** illustrates interconnected reaction pathways that lead to products present in the glyconitrile CRN, thus showing the network topology of its underlying chemistry.
**Figure 8** illustrates two different matrices of chemical input conditions designed to evaluate the influence of different variables on the glyconitrile CRN composition in CSTR.

### DETAILED DESCRIPTION

### Definitions

The term "*chemical reaction network*", abbreviated as CRN, is understood as a set of reactions interconnecting a set of products based on a set of reactants, a set of reactant concentrations and a set of reaction conditions, such as for example pH or temperature. The set of reactants, set of reactant concentrations and/or the set of reaction conditions may be defined and fixed at the beginning, or they may be varied over time depending on the specific chemical reaction network to be implemented. With regard to the particular chemical reaction network described herein, the terms "*glyconitrile-based chemical reaction network*", "*glyconitrile chemical reaction network*", "*glyconitrile CRN*" and "*chemical reaction network based on glyconitrile*" are used interchangeably.

In the context of the present invention, the terms "*compound(s)*" and "*substance(s)*" may be used interchangeably.

In the context of the present invention, the terms "*reactant(s)*" and "*reagent(s)*" may be used interchangeably.

The term "*starting compound*" or "*starting compounds*" is understood herein as those compounds that are used as a starting material to generate the glyconitrile chemical reaction network and may include reactive species, such as glyconitrile as well as any auxiliary reagents such as the salt. The term "*set of starting compounds*" is meant to refer to one or more different starting compounds that may be provided together, individually or in any combination at any time of the implementation of the chemical reaction network. For example, in the batch mode, the set of starting compounds may be mixed together in a single reactor to generate the chemical reaction network. Or in the continuous mode for example, the set of starting compounds may be added to the reactor all together, individually or partially mixed. For every set of starting compounds, there will be used an initial concentration to start the generation of the chemical reaction network.

The term "*initial concentration*", "*input concentration*" or "*starting concentration*" is understood herein as the concentration of a starting compound that may be a compound or reagent, or a mixture of compounds or mixtures of reagents, which is used at the start of implementing and/or generating the chemical reaction network.

The term "*input solution*" or "*input factor*" is understood as a solution of a set of starting compounds, such as compound(s) or reagent(s), that is used to start or influence the chemical reaction network. In the context of continuous reaction systems, the input solution or input factor is a solution a set of starting compounds, such as compound(s) or reagent(s), that enters the reaction reactor by the inlet channel(s), not only at the start but also during the ongoing reaction, if an additional amount of compound(s) or reagent(s) should be added to vary the chemical reaction network.

The term "*outflow*" in the context of continuous reaction systems is understood as the outgoing reaction solution or output composition that exits the output channel.

The term "*metabolomics*" is understood as the systematic study of the unique chemical fingerprints that specific cellular processes leave behind. In the present invention, "*metabolomics*" refers to the study of the unique chemical fingerprints of the glyconitrile CRN using different analytical techniques that may be combined in any manner. For example, possible analytical techniques that may be used here are ¹H-NMR, ¹³C-NMR, ¹⁵N-NMR, HPLC, Ultra HPLC, LC, HPLC-MS, Ultra HPLC-MS, LC-MS, MS, MS/MS, GC, GC-MS. Other analytical techniques not commonly used in the metabolomics field, such as FTIR and UV-Vis, together with pH measurements, temperature monitoring, or chemical input concentration monitoring, can also be used to control or monitor CRNs. Further, these techniques may be combined with any bioinformatics tool, such as any compound libraries, automatic MS data interpretation tools, prediction tools, data processing tools or artificial intelligence tools.

The term "*batch mode*" in the context herein is understood as a reaction mode in which a defined set of reactants with a defined set of reactant concentrations is reacted under a defined set of reaction conditions.

The term "*continuous mode*" is understood herein as a continuous system, wherein the input of reactants and the corresponding output of reaction products is continuous over time, and wherein the set of reactants, the set of reactant conditions and/or the set of reaction conditions may be varied during the reaction.

The term "*principal component analysis (PCA)*" is known in the field of data processing and represents a linear dimensionality reduction technique. The data is linearly transformed onto a new coordinate system such that the directions (principal components) capturing the largest variation in the data can be easily identified.

The term "*matrix of conditions*" is meant herein as a matrix of the set of reaction conditions that is applied to the chemical reaction network. The type of the matrix will depend on the total number of reaction conditions.

The term "*heatmap*" is understood herein as a 2-dimensional data visualization technique that represents the magnitude of individual values, here individual reaction products or reaction intermediates, within the dataset as a colour.

The term "*cluster*" is understood herein as a group of similar reaction products, reaction compounds or reaction intermediates that respond coherently to the applied reaction conditions and so their synthetic pathways are interconnected.

The term "*dendrogram*" in the present invention is understood as a diagram representation of the data in form of a tree. Each "*leaf*" of this tree diagram may represent one cluster as described above.

The term "*dataset(s)*" is understood herein as a variety of data that depends on the input variables, the reaction conditions and the analytical techniques used to determine the reaction output. The dataset(s) may then be graphically illustrated, for example in form of a heatmap or a PCA, allowing thus the identification of specific reaction patterns.

The term "*water-miscible organic solvent*" is any organic solvent that is at least partially miscible with water, preferably completely miscible with water.

### Glyconitrile-based chemical reaction network

Studying chemistry-based CRNs holds great potential. Systems with the adequate balance between dynamic complexity and tuneability are required for this purpose, which implies being able to control the size, topology and diversity of the CRN by chemical inputs. This requires also an efficient monitoring with high reproducibility of the internal composition dynamics by following the evolution of a high number of chemical species, which poses a tremendous analytical challenge. A combination of different and powerful analytical techniques, some of them commonly used in metabolomics, such as liquid chromatography (LC), mass spectrometry (MS) and nuclear magnetic resonance (NMR), or infrared (IR) spectroscopy are needed to tackle this challenge.

The present invention refers to a new chemical reaction network based on glyconitrile, its implementation and a method of using said chemical reaction network. In particular, the glyconitrile CRN of the present invention refers to a set of interconnected compounds and reactions between them, all generated from glyconitrile, which at temperatures ≥ 20 °C and a pH > 7 displays the required complexity, hyperdimensionality and non-linearity desired for a CRN. The glyconitrile CRN of the present invention is non-linearly dependent on conditions such as temperature, base or glyconitrile concentration, and can be easily influenced by these chemical inputs, and also by others, to change its internal state and, thus, yields different chemical outcomes.

In one aspect, the invention refers to a glyconitrile chemical reaction network generated from (a) a glyconitrile solution at a pH > 7; and to a method for implementing a glyconitrile chemical reaction network generated from a set of starting compounds comprising (a) glyconitrile; (b) a salt; and (c) a base.

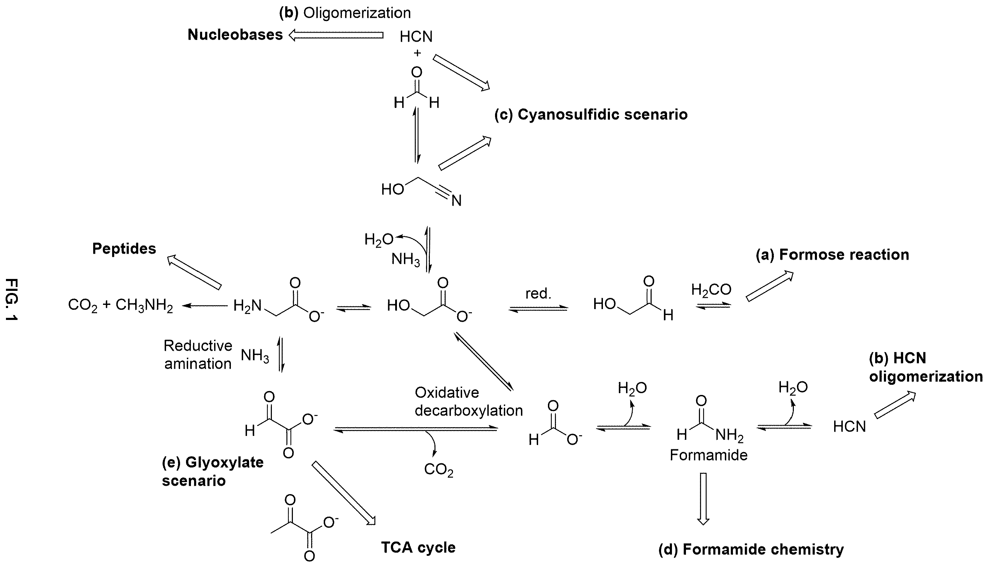

Glyconitrile is a relatively simple compound, that is also known as hydroxyacetonitrile, cyanomethanol or formaldehyde cyanohydrin. It is formed from formaldehyde and hydrogen cyanide and may thus be used as a temporary form for these two reagents. Glyconitrile is stable under acidic conditions and may polymerise under basic conditions. It is liquid at room temperature and dissolves readily in water, ethanol or ether. Particularly in aqueous media, glyconitrile is the result of a fast and thermodynamically favourable condensation process between HCN and formaldehyde. Hence, glyconitrile is a very reactive derivative that additionally interconnects different prebiotic synthetic pathways. An example of those different pathways based on the central position of glyconitrile is illustrated in Fig. 1. Glyconitrile may be a source to obtain access to a variety of compounds, such as, for example, amino acids, peptides, nucleobases, carbohydrates or other relevant molecules. For example, glyconitrile is a key intermediate species in the cyanosulfidic synthetic scheme towards nucleotides, amino acids and some lipid precursors (e.g., glycerine). By reduction into glycoaldehyde it also provides the main precursors of the formose reaction system, which leads to the formation of carbohydrates. Finally, it can also be connected with the formamide-based prebiotic reaction system, and with the glyoxylate prebiotic reaction system that leads to many of the citric acid cycle metabolites. Hence, glyconitrile appears to be a key precursor for a large variety of prebiotic molecules and, upon addition of other chemical inputs, potential drug candidates.

In the present invention, the glyconitrile CRN may use glyconitrile as such or as a solution. Due to the dilution factor and potential risks of its decomposition into formaldehyde and hydrogen cyanide, it is preferred to provide glyconitrile in solution. The solution may be selected from an aqueous solution, an ethereal solution, an alcoholic solution or mixtures thereof. The ethereal solution may be formed from diethylether. The alcoholic solution may be formed from ethanol. Preferably, an aqueous solution is used.

The aqueous glyconitrile solution may be formed from only water, or may be formed from water and a water-miscible organic solvent. The water-miscible organic solvent may be selected from the group consisting of ethanol, acetonitrile, tetrahydrofuran, acetone, or mixtures thereof. A water-miscible organic solvent may be added to improve solubility of the forming products in the generated CRN. The water-miscible solvent may be added in an amount of up to 10%, preferably in a range from 0 to 10%, more preferably from 0-7.5%, even more preferably from 0 to 5% (v/v).

Preferably, the aqueous glyconitrile solution is formed from only water.

The use of an aqueous glyconitrile solution has the advantage that harmful and expensive organic solvents may either mainly or completely be avoided. Moreover, using water as a solvent is environmentally friendlier and provides a broad temperature range to work with and better temperature stability during the reactions.

The initial (starting) concentration of glyconitrile in the aqueous solution may be more than 250 mM, preferably from 250 mM to 1 M, more preferably from 280 mM to 750 mM, even more preferably from 300 mM to 620 mM, and even more preferably from 320 to 520 mM. Within these ranges, glyconitrile can form various compounds of interest, while undesired polymerisation and the risks thereof are avoided.

In the present invention, the glyconitrile CRN comprises additionally a salt as a starting compound. In one aspect of the invention, the salt may be selected from the group consisting of alkali halides, alkaline earth metal halides, or mixtures thereof. Preferably, the salt may be selected from the group consisting of alkali halides, or mixtures thereof.

In one aspect, the alkali halides may be selected from the group consisting of lithium halide, sodium halide, potassium halide, rubidium halide, caesium halide, ammonium halide, or mixtures thereof. Alternatively, the alkali halides may be selected from the group consisting of alkali fluorides, alkali chlorides, alkali bromides, alkali iodides, or mixtures thereof. Alternatively, the alkali halides may be selected from the group consisting of lithium fluoride, lithium chloride, lithium bromide, lithium iodide, sodium fluoride, sodium chloride, sodium bromide, sodium iodide, potassium fluoride, potassium chloride, potassium bromide, potassium iodide, rubidium fluoride, rubidium chloride, rubidium bromide, rubidium iodide, caesium fluoride, caesium chloride, caesium bromide, caesium iodide, ammonium fluoride, ammonium chloride, ammonium bromide, ammonium iodide, or mixtures thereof. Preferably, the alkali halides may be selected from the group consisting of lithium fluoride, lithium chloride, lithium bromide, lithium iodide, sodium fluoride, sodium chloride, sodium bromide, sodium iodide, potassium fluoride, potassium chloride, potassium bromide, potassium iodide, or mixtures thereof. More preferably, the alkali halide is sodium chloride.

In one aspect, the alkaline earth metal halides may be selected from the group consisting of beryllium halide, magnesium halide, calcium halide, strontium halide, barium halide, or mixtures thereof. Alternatively, the alkaline earth metal halides may be selected from the group consisting of alkaline earth metal fluorides, alkaline earth metal chlorides, alkaline earth metal bromides, alkaline earth metal iodides, or mixtures thereof. Alternatively, the alkaline earth metal halides may be selected from the group consisting of beryllium fluoride, beryllium chloride, beryllium bromide, beryllium iodide, magnesium fluoride, magnesium chloride, magnesium bromide, magnesium iodide, calcium fluoride, calcium chloride, calcium bromide, calcium iodide, strontium fluoride, strontium chloride, strontium bromide, strontium iodide, barium fluoride, barium chloride, barium bromide, barium iodide, or mixtures thereof. Preferably, the alkaline earth metal halides may be selected from the group consisting of magnesium bromide, magnesium iodide, calcium fluoride, calcium chloride, calcium bromide, calcium iodide, or mixtures thereof.

The initial (starting) concentration of the salt in the glyconitrile CRN may be more than 100 mM, for example from 100 mM to 1 M, preferably from 100 mM to 600 mM, more preferably from 150 mM to 500 mM, even more preferably from 200 mM to 500 mM, and even more preferably from 300 to 450 mM. Most preferably, the initial (starting) concentration of the salt in the glyconitrile CRN is about 400 mM. Within these ranges, the salt provides an effective ionic strength and thus provides excellent solubility for the starting materials as well as for the compounds to be formed.

In the present invention, the glyconitrile CRN comprises also a base as a starting compound. In one aspect of the invention, said base is present in amounts sufficient to provide a pH value of more than 7, preferably ≥ 8, more preferably ≥ 9, more preferably ≥ 10, and even more preferably > 10. In another aspect of the invention, the base is present in amounts sufficient to provide a pH value of 8 to 14, preferably 9 to 14, and even more preferably from 10 to 14.

Bases that are suited to provide a pH value of more than 7 and up to 14 are known and may be selected from inorganic bases or organic bases. In one aspect of the invention, the base is selected from the group consisting of alkali hydroxides, alkaline earth metal hydroxides, ammonia, mono alkyl amines, dialkyl amines, trialkyl amines, alkali phosphates, alkaline earth metal phosphates, alkali carbonates, alkaline earth metal carbonates, or mixtures thereof. Alkali hydroxides may refer to lithium hydroxide, sodium hydroxide, potassium hydroxide, rubidium hydroxide or caesium hydroxide. Alkaline earth metal hydroxides may refer to magnesium hydroxide, calcium hydroxide, strontium hydroxide or barium hydroxide. Ammonia may refer to the gas ammonia or to aqueous ammonia solutions. Mono alkyl amines refer to methyl amine, ethyl amine, propyl amine, isopropyl amine or guanidine. Dialkyl amines refer to dimethyl amine, diethyl amine, dipropyl amine, or diisopropyl amine. Trialkyl amines refer to trimethyl amine, triethyl amine, tripropyl amine, triisopropyl amine, 1,4-diazabicyclo[2.2.2]octane (DABCO), diisopropylethyl amine (DIPEA).

In one aspect, the alkali phosphates may refer to monophosphates derived from orthophosphate, hydrogen phosphate or dihydrogenphosphate, to diphosphates, to triphosphates, to tetraphosphate or to cyclic polyphosphates (also called metaphosphates). Preferably, the alkali phosphate is a monophosphate selected from the group of lithium monophosphate, sodium monophosphate, potassium monophosphate, rubidium monophosphate, caesium monophosphate, or mixtures thereof.

In one aspect, the alkaline earth metal phosphates may refer to monophosphates derived from orthophosphate, hydrogen phosphate or dihydrogenphosphate, to diphosphates, to triphosphates, to tetraphosphate or to cyclic polyphosphates (also called metaphosphates). Preferably, the alkaline earth metal phosphate is a monophosphate selected from the group of magnesium monophosphate, calcium monophosphate, strontium monophosphate, barium monophosphate, or mixtures thereof.

In one aspect, the alkali carbonates may be selected from the group consisting of lithium carbonate, sodium carbonate, potassium carbonate, rubidium carbonate, caesium carbonate or ammonium carbonate, lithium bicarbonate, sodium bicarbonate, potassium bicarbonate, rubidium bicarbonate, caesium bicarbonate, ammonium bicarbonate, or mixtures thereof. Preferably, the alkali carbonates may be selected from the group consisting of lithium carbonate, sodium carbonate, potassium carbonate, lithium bicarbonate, sodium bicarbonate, potassium bicarbonate, or mixtures thereof.

In one aspect, the alkaline earth metal carbonates are alkaline earth metal bicarbonates. Preferably, said alkaline earth metal bicarbonates may be selected from the group consisting of magnesium bicarbonate, calcium bicarbonate, strontium bicarbonate, barium bicarbonate, or mixtures thereof.

In another aspect, the base is selected from the group consisting of alkali hydroxides, alkaline earth metal hydroxides, trialkyl amines, or mixtures thereof. Preferably, the base is selected from the group consisting of alkali hydroxides, alkaline earth metal hydroxides, or mixtures thereof, more preferably alkali hydroxides.

In yet another aspect, the base is selected from the group consisting of lithium hydroxide, sodium hydroxide, potassium hydroxide, rubidium hydroxide, caesium hydroxide, magnesium hydroxide, calcium hydroxide, strontium hydroxide, barium hydroxide, ammonia, aqueous ammonia solutions, methyl amine, ethyl amine, propyl amine, isopropyl amine, guanidine, dimethyl amine, diethyl amine, dipropyl amine, diisopropyl amine, trimethyl amine, triethyl amine, tripropyl amine, triisopropyl amine, 1,4-diazabicyclo[2.2.2]octane (DABCO), diisopropylethyl amine (DIPEA), or mixtures thereof. Preferably, the base is selected from the group consisting of lithium hydroxide, sodium hydroxide, potassium hydroxide, magnesium hydroxide, calcium hydroxide, methyl amine, ethyl amine, propyl amine, isopropyl amine, dimethyl amine, diethyl amine, dipropyl amine, diisopropyl amine, trimethyl amine, triethyl amine, tripropyl amine, triisopropyl amine, 1,4-diazabicyclo[2.2.2]octane (DABCO), diisopropylethyl amine (DIPEA), or mixtures thereof. More preferably, the base is selected from the group consisting of sodium hydroxide, potassium hydroxide, magnesium hydroxide, calcium hydroxide, trimethyl amine, triethyl amine, diisopropylethyl amine (DIPEA), or mixtures thereof. In a particular aspect, the base is selected from the group consisting of sodium hydroxide, potassium hydroxide, or mixtures thereof.

The initial (starting) concentration of the base in the glyconitrile CRN is at least sufficient to provide a pH value of more than 7, preferably from 8 to 14. In a preferred aspect, the initial concentration of the base may be more than 30 mM, preferably from 30 mM to 750 mM, more preferably from 50 mM to 600 mM, even more preferably from 50 mM to 500 mM, and even more preferably from 50 to 450 mM. Within these ranges, the base provides a basic pH environment that allows the formation and implementation of the glyconitrile CRN and thus the formation of various compounds of interest in one single pot.

The initial (starting) concentration of the base may be used to direct or modulate the glyconitrile CRN towards specific outcomes. For example, it has been observed that the glyconitrile CRN profiles and the reaction dynamics of chemical species may depend on the initial pH.

In another aspect of the invention, the glyconitrile CRN is implemented at temperatures of not more than or equal to 100°C. Preferably, the temperature is maintained between 20 °C and 100 °C, more preferably between 20 °C and 90 °C, more preferably between 30 °C and 80 °C, and most preferably between 40 °C and 70 °C. In an alternative aspect of the invention, the temperature may be selected from the group consisting of 40 °C, 45 °C, 50 °C, 55 °C, 60 °C, 65 °C, or 70 °C. In one particular aspect of the invention, the selected temperature is maintained constant over the whole time. In another particular aspect, the glyconitrile CNR may be submitted to an increasing or decreasing temperature gradient, wherein the selected starting temperature is then gradually increased or decreased over a fixed step of time. For example, (i) a starting temperature is selected from one the ranges mentioned above, (ii) then maintained for a given set time, such as between 5 and 20 minutes, (iii) after completing the set time of step (ii) the composition is analysed, (iv) then the temperature is increased or decreased by e.g. 3 K, (v) then the temperature is maintained again for the same set time as in step (ii), (vi) after completing the set time of step (v) the composition is analysed, and then steps (iv) to (vi) may be repeated again until reaching a predetermined end temperature.

The temperature may have an influence on the outcome of the glyconitrile CRN. It has been observed that the glyconitrile CRN is sensitive to the applied temperature and its complexity increases with increasing temperature.

In one aspect of the invention, the glyconitrile CRN once started, has a run time of from 1 minute up to 91 minutes. The run time is the time during which the glyconitrile CRN is implemented. In some instances, the run time may coincide with the sampling time, at which samples are taken to analyse and determine the current composition of the glyconitrile CRN. In other instances, the run time may include more than one sampling times to allow for more than one samples to be taken and monitor the implementation and development of the glyconitrile CRN. Since the glyconitrile CRN has some latency once it is started, the first minute of run time is neglected and in the case of a continuous mode, the outcome of this first minute is discarded. Samples may then be taken as desired, for example preferred run times may be 1 minute, 19 minutes, 37 minutes, 55 minutes, 73 minutes or 91 minutes. Other examples of the run time for the investigation of certain conditions of the glyconitrile CRN may be 15 minutes, 30 minutes, 45 minutes, 60 minutes or 90 minutes. These run times may be sufficient in order to achieve a glyconitrile CRN with a steady state. The skilled person may adjust the run time depending on the means he has available to implement and/or analyse the glyconitrile CRN.

However, in other aspects of this invention, the run time may be longer than 91 minutes. For example, the run time may be up to 300 minutes, for example, to investigate the dynamic behaviour of the glyconitrile system.

The glyconitrile CRN and its implementation can be performed in batch or in continuous mode. In either mode, the glyconitrile CRN as described above shows a high reproducibility that can be shown by pH measurements as well as by HPLC-MS data obtained from sampling. This includes the use of the glyconitrile CRN in batch or continuous mode or processes to implement the glyconitrile CRN in batch or continuous mode. In the present invention, method of implementation is the same as process for implementation.

Irrespective of its mode of implementation, the method of implementing the glyconitrile CRN comprises the steps of (a) sample preparation, (b) sample analysis, and (c) compound identification. Additionally, the method of implementation may comprise a step of (b.1) data acquisition and data extraction and a step of (b.2) data processing. For every step, adequate means are provided in order to perform the steps and implement the glyconitrile CRN.

The step of (a) sample preparation may comprise, among others, the preparation of the starting compound solution or solutions, either in situ or in form of a stock solution, the provision of a reactor for either batch or continuous mode, and/or any necessary means for keeping the glyconitrile CRN on-going or acquiring samples therefrom.

The step of (b) sample analysis may comprise, among others, the steps of taking a sample from the glyconitrile CRN, preparing the sample for analysis, analysing a sample, providing data acquisition and data extraction, and/or performing data processing. In some aspects, a physical sample is taken from the glyconitrile CRN for sample analysis. In other aspects, sample analysis may be performed in situ, without the need to take a physical sample. In still other aspects, sample analysis is done in situ as well as by taking a physical sample from the glyconitrile CRN.

The step of (c) compound identification may comprise, among others, converting the sample analysis results into its respective chemical structures and determine the quantity or concentration of each identified compound.

Depending on batch mode or continuous mode, the means for sample preparation and sample analysis may differ, whereas the means for data acquisition and data extraction, the means for data processing, and the means for compound identification may be the same or different in the batch and the continuous mode.

Means for sample preparation may comprise the starting input solutions of each starting compound, prepared either in situ or prepared in form of a stock solution; the reactor in which the glyconitrile CRN is prepared, which may be adapted either for batch mode or for continuous mode; and/or an outflow recipient when working in continuous mode. For example, in continuous mode, the means for sample preparation may, along with other means for sample preparation, comprise means for a continuous reaction system having at least one inlet channel, preferably from 1 to 8 inlet channels.

Means for sample analysis, or simply means for analysis, may comprise any device and/or apparatus for performing UV-Vis, ¹H-NMR, ¹³C-NMR, ¹⁵N-NMR, HPLC, Ultra HPLC, LC, HPLC-MS, Ultra HPLC-MS, LC-MS, MS, MS/MS, GC, GC-MS, or FTIR; and/or any device for controlling and/or monitoring the glyconitrile CRN, such as pH sensor, temperature sensor, or chemical input concentration monitoring.

Means such as any bioinformatic tool, such as any compound libraries, automatic MS data interpretation tools, prediction tools, data processing tools or artificial intelligence tools may be considered as means for sample analysis as well as means for compound identification, since they may provide the means for data acquisition, data processing and compound identification in one single entity.

All the means described herein may be combined in any manner to form together a device configured for implementing a glyconitrile chemical reaction network according to the invention.

For example, this may be a device configured for implementing a glyconitrile chemical reaction network according to any aspect described herein comprising (i) means for the preparation of the glyconitrile chemical reaction network, (ii) means for sampling the glyconitrile chemical reaction network composition one or more times at defined time points providing one or more sample datasets, (iii) means for analysing the one or more sample datasets, including means for data acquisition and data extraction, providing one or more analysis datasets, (iv) means for data processing of the obtained one or more analysis datasets, and (v) means for compounds identification.

In one aspect of the invention, the glyconitrile CRN is implemented in batch mode. Any recipient may be used for batch mode, but it is preferable to use small volumes, such as 1 mL, in order to avoid concentration gradients at any point in the solution. For example, the volume used in batch mode may range from 0.1 mL to 2 mL, preferably from 0.2 mL to 1 mL. In one aspect, test tubes can be used. Batch mode means that the input conditions cannot be recursively changed, and the dependence of the glyconitrile CRN composition with the input conditions is established by analysing different reactions with different concentrations of the three reagents and at different temperatures.

In another aspect of the invention, the glyconitrile CRN is implemented in continuous mode, for example in a continuously stirred tank reactor (CSTR), where various inlet channels can be used to modify different chemical input variables. Hence, in the CSTR mode it is possible to continuously change input conditions and to analyse the output composition after such a change, allowing to influence the glyconitrile CRN state in a recursive way. A CSTR is used having 1 to 10 input channels and 1 to 3 output channels, preferably 1 to 8 input channels and 1 output channel. Every inlet channel may supply then different solutions containing one or more of the starting compounds in different concentrations. The reactor is preferably equipped with a magnet and deposited on a temperature-controlled stirring plate. Each inlet channel may be activated at the same time or at different times. Each inlet channel may be activated with a specific flow rate that may depend on the reactor size and the desired residence time. Each inlet channel may be operated with an individual flow rate, or all inlet channels may be operated with the same flow rate. The higher the flow rate, the shorter the residence time. The flow rate may thus be adapted according to the desired residence time or vice versa for each input channel. In one preferred aspect, the flow rate is selected from a range of from 0 to 34 µL/min, preferable from 0 to 17 µL/min. In another preferred aspect, the residence time is selected from a range of from 3 minutes to 2 hours, preferably from 3 minutes to 30 minutes. During an initial phase, that may range from 30 seconds to 3 minutes, preferably from 30 seconds to 1 minute, the output channel is pulled to waste, and the outflow is then collected every 2 min in a 1.5 mL Eppendorf tube placed in an ice bath to allow monitoring the glyconitrile CRN over time. The different channel inflows may be used to determine and to steer the reactor conditions. The inflow of the input channels can be recursively modified over the experiment, defining different patterns, even over time (including oscillatory ones), of the different chemical inputs. This offers advantages for exploration and optimization of multiple reaction pathways within the glyconitrile CRN, such as the possibility to feed the reactor with changing input concentrations of the starting compounds that affect the network composition, continuously shifting its steady state to new measurable situations. Hence, this allows studying the interconversion of the different species form in the glyconitrile CRN, and thus studying multiple pathways in the glyconitrile CRN, which eventually will not only provide the best synthetic pathway, but also the most robust synthetic pathway, since the multiple pathways in the glyconitrile CRN compete against each other. Additionally, this may open access to new synthetic routes that would not have been considered with the traditional retrosynthetic approach in organic chemistry.

In either mode, the glyconitrile CRN may be controlled and monitored by several ways and several means or methods. For example, temperature control is carried out to maintain the temperature constant during the whole run time. Additionally, a pH sensor may be present to monitor the pH value and the hydroxide concentration over time. This allows for example to estimate the rate of consumption of hydroxyl ions and determine the kinetics with respect to the base consumption. In this respect, it has been found that there is a direct dependence between the concentration of glyconitrile and the hydroxide consumption. The higher the glyconitrile concentration, the quicker the hydroxide consumption, meaning a quicker drop of the pH of the system, which provokes very important compositional changes in the glyconitrile CRN and provides access to a large variety of compounds.

Another monitoring method or monitoring and analysis means is the ¹H-NMR technique, which can be applied to observe the glyconitrile concentration over time. For example, the disappearance of the glyconitrile ¹H-NMR singlet at 4.42 ppm, with respect to the signal intensity of the internal standard 3-(trimethylsilyl)-2,2,3,3-tetradeuteropropionic acid (TMSP), may be followed for quantification at each particular reaction time. The data so obtained allows estimating the consumption of glyconitrile, measured in nM per minute, and determine the reaction rate for a glyconitrile CRN under specific conditions. These reaction rates may then give an indication of the time it will take to reach a new steady state when changing a given concentration in the input chemical composition.

The composition of each glyconitrile CRN and its change over time may be determined or monitored further by UV-Vis spectra, LC-MS and/or HPLC-MS, or FT-IR spectroscopy, or any combination thereof. The resulting spectra and chromatograms will change over time and depend on the initial NaOH concentration. This may be used to determine reproducibility. Using additionally tandem mass spectrometry, also indicated as MS/MS, or using the combination of the exact mass (m/z) and the corresponding retention time (RT value), different species can be identified within the glyconitrile CRN. Hence, using different combinations of analysis means, it becomes possible to identify different chemical species, for example based on their kinetic/dynamic profile or their MS/MS spectra, and even monitor their specific relative abundance at each reaction time, so to follow the dynamics of multiple species at the same time. Hence, it is possible to follow the chemical behaviour and identify reactive intermediates and their transformations, as well as a fingerprint of the glyconitrile CRN output composition based on the relative MS intensities for a given time.

In the present invention, any analytical method, or monitoring method, or controlling method is also an analysis means, monitoring means or controlling means, respectively.

### Applications of glyconitrile CRNs

The glyconitrile CRN of the present invention offers a very broad chemical and structural diversity, because glyconitrile is a versatile precursor that involves both nitrogenous and oxygenous reactive functional groups. Hence, it leads not only to linear sugars but, at the same time, can also produce amino acids and derivatives thereof, their linear oligomers, aminonitrile and derivatives thereof, purine and pyrimidine nucleobases, aliphatic and aromatic heterocycles, and more. In general, the glyconitrile network of the present invention leads to a wide variety of organic structures with high diversity of functional groups, which enhances the possibility of finding compounds with relevant pharmaceutical activities, especially if one or more additional chemical inputs are present, preferably when these additional chemical inputs have structures that are considered precursors of drug lead structures.

Additionally, the glyconitrile CRN provides chemical compositions that enable many alternative analytical ways to perform a read out thereof, because of the formation and presence of various functional groups. This includes UV-Vis and FT-IR spectroscopy monitoring, in addition to HPLC-MS or LC-MS or GC-MS. For example, the samples may show complex patterns of FT-IR peaks that reproducibly change with the input conditions, and patterns of absorption bands in the UV-Vis region up to 500 nm owing to the presence of aromatic compounds in the mixture.

The glyconitrile CRNs of the present invention can be used to expand experimental glyconitrile CRNs towards the simultaneous formation of a variety of molecules with potential pharmacological properties, favouring both the screening of their biological activity and the inference of optimal routes to synthesize them. Hence, one aspect herein refers to a process for preparing molecules with potential biological and/or pharmacological properties, their salts, esters or metabolites thereof using a chemical reaction network comprising glyconitrile, comprising the steps of generating a glyconitrile CRN, analysing the composition after a set time, identifying one or more desired molecules with potential biological and/or pharmacological properties, and determine the synthetic intermediates thereof to identify synthetic pathways. Alternatively, it refers to a method for preparing molecules with potential biological and/or pharmacological properties, their salts, esters, or metabolites thereof using a chemical reaction network comprising glyconitrile, comprising the steps of generating a glyconitrile CRN, analysing the composition after a set time, identifying one or more desired molecules with potential biological and/or pharmacological properties, and determine the synthetic intermediates thereof to identify synthetic pathways. Alternatively, it refers to the use of a glyconitrile CNR for preparing molecules with potential biological and/or pharmacological properties, their salts, esters, or metabolites thereof using a chemical reaction network comprising glyconitrile, comprising the steps of generating a glyconitrile CRN, analysing the composition after a set time, identifying one or more desired molecules with potential biological and/or pharmacological properties, and determine the synthetic intermediates thereof to identify synthetic pathways. The advantage of this aspect is that besides the targeted molecules, the interaction with the highly reactive intermediates of the glyconitrile CRN will enable the preparation of multiple derivatives of such drugs in a one-pot scheme, and not have to run single experiments for each compound and intermediate. This aspect comprises also the use of means for identifying said molecules and their respective intermediates, means for screening their biological activity, means for identifying their synthetic formation pathways. Eventually, the use of the glyconitrile CRN will allow determining those conditions that may maximize their production within the glyconitrile CRN.

This is based on the fact that glyconitrile is the result of a fast and thermodynamically favourable addition of HCN to formaldehyde. This is an equilibrium process that can be reverted under certain conditions and is relevant because HCN is known to oligomerize yielding nucleobases while formaldehyde leads to sugars in the so-called formose reaction. Hence, glyconitrile is a very reactive species that can engage in different synthetic and decomposition pathways, occupying a central position in the cyanosulfidic scenario that can give rise to various amino acids, activated nucleotides and lipid precursors through organic synthesis.

The glyconitrile CRN of the present invention, the implementation of the glyconitrile CRN of the present invention and the use of the glyconitrile CRN of the present invention have the advantage that glyconitrile transformations occur in one pot, establishing thus a network behaviour, and form a variety of biologically relevant products in that network, including amino acids, such as glycine, diglycine and triglycine derivatives; purine and pyrimidine derivatives thereof; nucleobases such as 3-methyladenine and 4-amino-2-methylpyrimidine-5-carbonitrile; nucleoside analogues such as cytarabine; nucleotide precursors such as RAO and AAO; and other derivatives with relevant biological activities thereof.

A non-exhaustive list of observed compounds, shown in Fig. 2, includes: (A) hexamethylenetetramine, (B) iminodiacetic acid, (C) N-(2-acetamido)iminodiacetic acid, (D) 3-methyladenine, (E) 4-amino-2-methylpyrimidine-5-carbonitrile, (F) isophoronediamine and (G) 1-(2-hydroxyethyl)-2,2,6,6-tetramethyl-4-piperidinol, (H) 3-cyclohexyl-1,1-dimethylurea, (I) cytarabine, (J) 2-[(2-hydroxyacetyl)amino]acetamide, (K) 2-amino-N-(2-hydroxyacetyl)acetamide, (L) 2-(hydroxymethyl)-1,4-dihydroimidazol-5-one, (M) [Bis(2-amino-2-oxoethyl)amino]acetic acid, (N) 2-[(2-amino-2-oxoethyl)-(cyanomethyl)amino]acetic acid, (O) 2-[(2-amino-2-oxoethyl)-(cyanomethyl)amino]acetamide, (P) 1(2H)-pyrazineacetamide,5-amino-3,6-dihydro-3-oxo-, (Q) 2-[[2-[(2-hydroxyacetyl)amino]acetyl]amino]acetic acid, (R) 2-((2-amino-2-oxoethyl)amino)acetic acid, (S) 2-[(carbamoylmethyl)amino]acetamide, (T) 2-[(2-amino-2-iminoethyl)amino]acetic acid, and the nucleotide precursors RAO and AAO.

Depending on the targeted molecule structures, one or more additional chemical inputs may be fed to the glyconitrile CRN system in order to favour the formation of specific molecules. For example, precursors of nucleotides appear in the system when glyceraldehyde and 2-aminooxazole are added as new starting compounds (in 10 mol%) with respect to glyconitrile. These two new starting compounds are considered core intermediates towards larger and more complex molecules in the cyanosulfide scenario (cf. Fig. 1) that may lead, besides said precursors of nucleotides, also to other amino acids, nucleotides as such or lipid precursors. Another new starting compound that can be added is glyoxal, which is a transient and highly reactive species that can been used as precursor of the antiviral drug favipiravir acting against RNA viruses such as influenza and SARS-CoV-2.

The advantage of connecting the glyconitrile CRN with the cyanosulfide scenario is that it opens the way to the synthesis of many biologically relevant species. Among nucleotides, amino acids and lipid precursors, other non-naturals species with potential biomedical activity may arise. Importantly, nucleosides and nucleotides constitute an important family of drugs. This family comprises many compounds acting against cancer, such as cytarabine, gemcitabine, mercaptopurine, azacytidine, cladribine, decitabine, fluorouracil, floxuridine, fludarabine, or nelarabine, most of them composed of a furanose sugar and a nucleobase. Other nucleoside analogues have antiviral activity, such as ribavirin and acyclovir. Since the glyconitrile CRN can be directed to the formation of nucleobases, the addition of the right sugar (and eventually exogenous nucleobases) as further chemical input may lead to the production of multiple analogues of these drugs.

The means for identifying said molecules and their respective intermediates are represented by the metabolomics procedure established in this invention. Said metabolomics procedure allows analysing the glyconitrile CRNs and thus help to find new structures generated. The tentative assignments of chemical structure to different species will be confirmed either with standards or with bioinformatic tools, such as a protocol of combination of SIRIUS and CFM-ID, to derive structures from mass fragmentation patterns (and vice versa).

Hence, said metabolomics procedure allows to build a "fingerprint" with the relative MS intensities for the compounds at each particular time in batch mode, and characterise the glyconitrile CRN output composition over time, for example in the form of heatmaps for a given initial pH value. This can also be extrapolated to the case in which the glyconitrile CRN is established in a CSTR. The heatmaps thus obtained represent on the x axis the different sampling times, while the y axis displays the different identified compounds as entries (RT_m/z). A colour code may be used to represent the relative MS intensities (after Z-score normalization) for the corresponding molecular ions. For example, the colour code may range from blue (lowest) to red (highest) or may be from white (lowest) to black (highest) as illustrated in Fig. 3. However, this is not so limited, and any possible colour code may be used. Such heatmaps will allow to show for each column a fingerprint of the composition at a given time, and the respective changes over time as the glyconitrile CRN progresses. Moreover, these heatmaps allow to group compounds in clusters with common dynamics. This clustering will be different depending on the input conditions and strongly correlates between the input composition and the resulting output compositions. Also, the clusters involve compounds that are quite diverse in chemical structure, according to their MS/MS fragmentation patterns. Without being bound to any theory, this suggests that derivatives belonging to the same cluster may be formed through related synthetic pathways, supporting the notion of a network interlinking them. This indicates that the glyconitrile CRN presents an intricate topology with highly interconnected nodes, which will result in and favour the hyperdimensionality and non-linearity necessary for future applications.

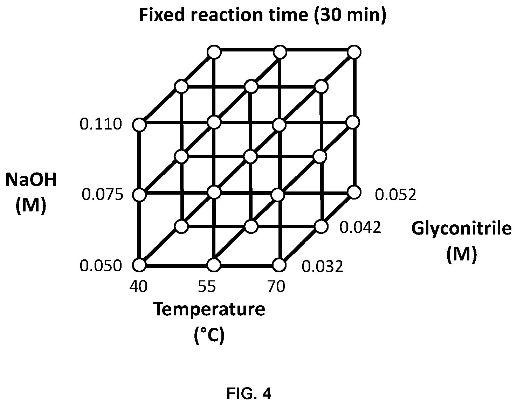

The glyconitrile CRN of the present invention and its analytical means allow also the mapping of its composition on different sets of chemical inputs. The analytical means may include a MS-based analytical platform to probe the network response to a broad range of experimental conditions, both in batch and in the CSTR. In batch mode, a matrix of conditions that evaluates three possible variables with three different values each may be used: glyconitrile concentration (for example: 0.52, 0.42 and 0.32 M), NaOH concentration (for example: 0.11, 0.075 and 0.05 M) and temperature (for example: 40, 55 and 70 °C) for a fixed reaction time of 30 minutes. Fig. 4 illustrates such a matrix in graphical form. Notwithstanding, any combination of variables may be used. This will allow to determine the influence for each factor based on the resulting complexity as well as the changes in the output compositions when the input variables are modified.

One approach to analyze, visualize and interpret the complexity of the data obtained from the glyconitrile CRN is to apply a non-supervised multivariate analysis, such as Principal Component Analysis (PCA). For this aim, the obtained data may be grouped either according to temperature-glyconitrile concentration pairs, or temperature-NaOH concentration pairs, so to obtain respective PCA score plots. Other groupings are also contemplated herein.

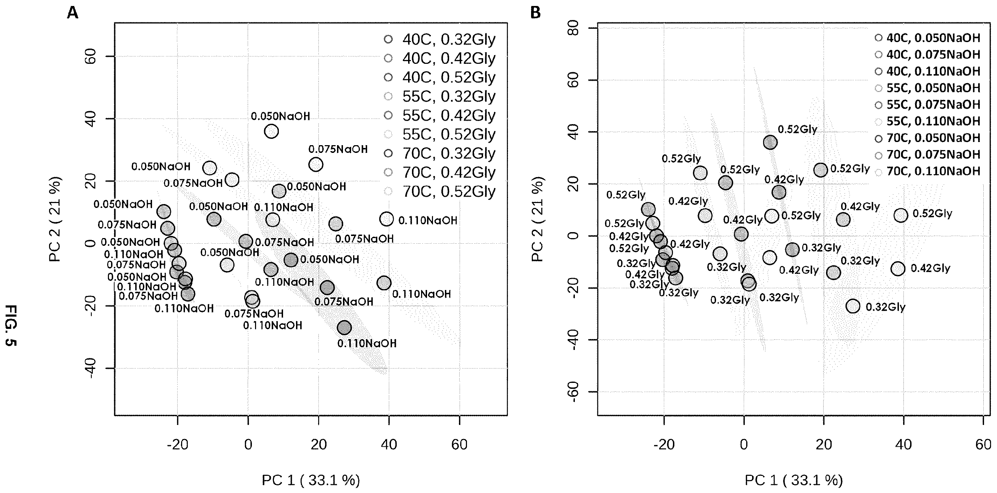

As observed in the present invention, the PCA score plots indicate that temperature as experimental condition has higher influence than glyconitrile or NaOH concentration. The temperature provides 33% of this variance, whereas glyconitrile and NaOH concentration explain 21% of the variance. PCA score plots of the present invention also illustrate that the samples obtained at 40 °C are grouped closer independently of the glyconitrile and NaOH concentration, while the samples obtained at 70 °C are more disperse, indicating a higher complexity for higher temperatures. This may be seen in the PCA score plot as illustrated in Fig. 5.

Within the matrix of conditions, it is possible to follow the evolution of the products over time, and thus to construct heatmaps that correlate the different observed species with the matrix of employed conditions. Such heatmaps may show a significant dependence of the composition on the temperature and the ratio between NaOH and glyconitrile concentrations and identify main clusters having patterns that will differ based on the experimental conditions used. Hence, combining PCA with heatmap/profile results allows identifying the influence of the reaction conditions and determining the respective complexity, hyperdimensionality and non-linearity of the glyconitrile CRN. For example, intensities of certain clusters may vary as a function of temperature. In some aspects, cluster intensities may increase with increasing temperature. In other aspects, cluster intensities may increase with decreasing temperature. And in still other aspects, cluster intensities may remain approximately constant over changing temperature. Separately or additionally, the cluster intensities and cluster formation may depend on the glyconitrile concentration and the pH (NaOH concentration), such as illustrated in Fig. 3. In some aspects, the effect of pH is higher than the effect of glyconitrile concentration. In other aspects the pH and the glyconitrile concentration do not have any effect. And in still other aspects, the glyconitrile concentration effect is higher than the effect from the pH value.

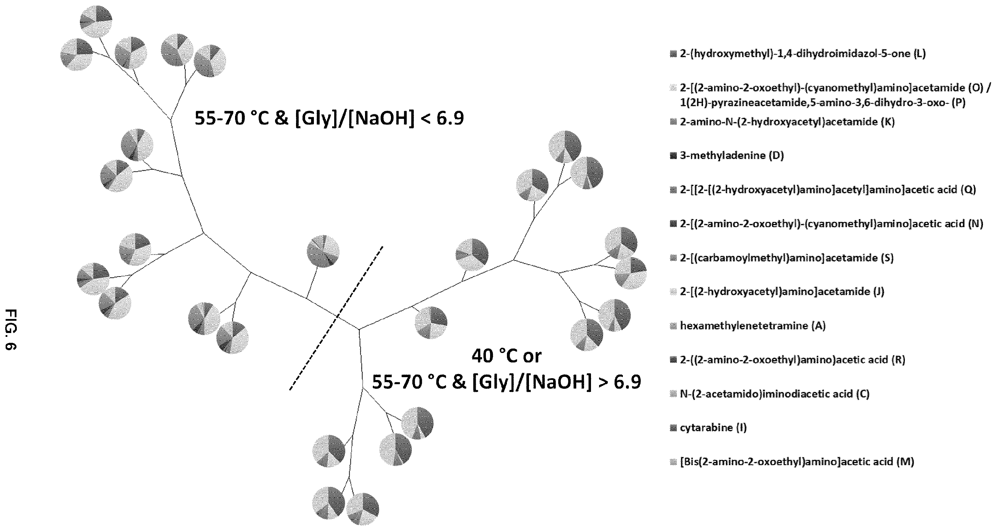

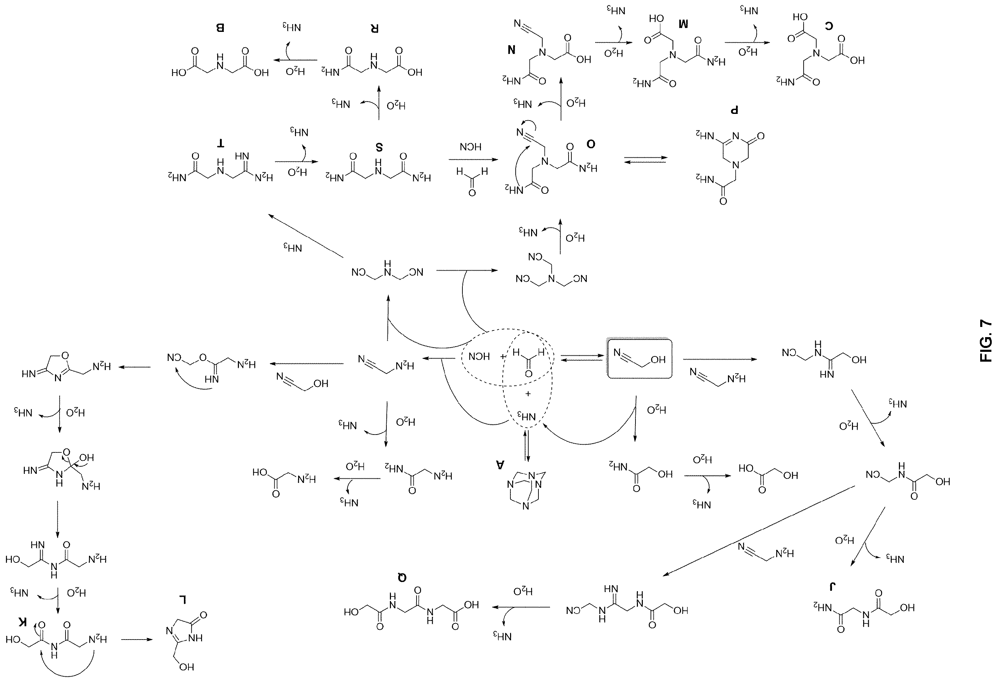

Another approach to analyze, visualize and interpret the complexity of the data obtained from the glyconitrile CRN is performing the identification of compounds present in the glyconitrile CRN at certain conditions as illustrated in Fig. 2 and then present the results in form of a dendrogram as illustrated in Fig. 6. The dendrogram represents a hierarchical clustering performed with the signals obtained from the batch glyconitrile CRN, where pie plots placed on the 'leaf' positions represent the normalized average product distributions of 13 of these compounds of Fig. 2. Each branch of the dendrogram in Fig. 6 arises as a result of the combination of the input factors. Hence, in Fig. 6, the left branches are characterized by medium to high temperature (55-70 °C) with an overall [Gly]/[NaOH] ratio < 6.9. Under these conditions, 2-[(2-amino-2-oxoethyl)-(cyanomethyl)amino]acetamide (O) (or its cyclic form 1(2H)-pyrazineacetamide,5-amino-3,6-dihydro-3-oxo- (P)) is the most represented species, followed by 2-[(carbamoylmethyl)amino]acetamide (S), 2-(hydroxymethyl)-1,4-dihydroimidazol-5-one (L) and 2-[(2-hydroxyacetyl)amino]acetamide (J). The samples obtained at 70 °C are also characterized by an increased abundance of 2-[(2-Amino-2-oxoethyl)-(cyanomethyl)amino]acetic acid (N). On the other hand, the right branches are characterized either by low temperature (40 °C), or medium to high temperature (55-70 °C) with an overall [Gly]/[NaOH] > 6.9. Under these conditions, 2-[(2-hydroxyacetyl)amino]acetamide (J) increased significantly with respect to the conditions in the left branches, as well as 2-(hydroxymethyl)-1,4-dihydroimidazol-5-one (L). This helps understanding how the starting input conditions of the glyconitrile CRN can then be adjusted for finding new compounds and which intermediates, precursors and/or metabolites may be present in the synthetic pathway. Hence, this provides the advantage that not only a target molecule may be identified, but also the best and most robust synthetic pathway leading thereto. For example, Fig. 7 shows proposed pathways based on the analysis and identification described above of compounds present in the system. Fig. 7 illustrates how these pathways are interconnected in a high degree, which eventually leads to a network topology that explains its subsequent non-linear dependency with the input conditions.

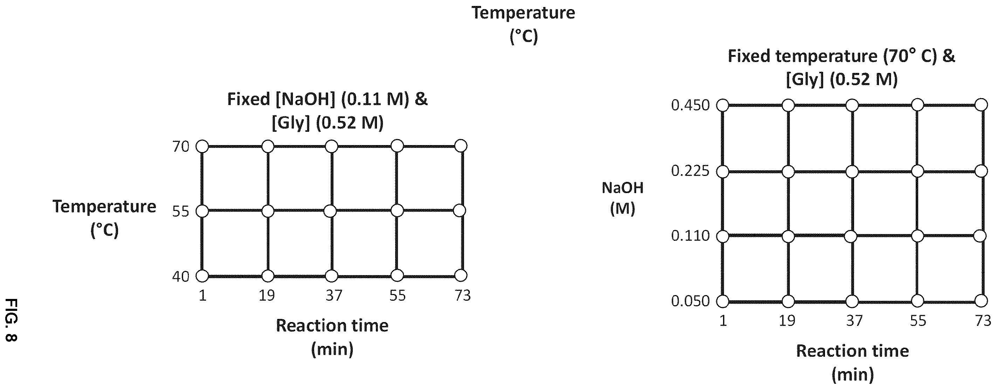

The CSTR systems have the additional advantage in that it is possible to continuously feed the reactor with changing input concentrations of the starting compounds that affect the network composition, thus shifting its steady state to a new measurable situation. Any variable may be varied, while others may be maintained constant. For example, temperature and/or pH may be varied, while the glyconitrile concentration is maintained constant. The analytical means will then allow to analyse the resulting mixtures at different intervals, introducing a third parameter (time), which will affect additionally the output compositions and the respective complexities. Matrices for the continuous mode may then be plotted as illustrated in Fig. 8. PCA analysis may be carried out to determine the influence on the variables and determine the divergence that will unfold over time. Again, it is possible to follow the evolution of the products over time, and thus to construct heatmaps that correlate the different observed species with the matrix of employed conditions and to identify specific clusters and their dependencies from temperature and or reagent concentrations.

The approach proposed herein can be seen as related to the classical combinatorial chemistry strategies to develop pharmaceutical drugs, with the difference that in CRNs they are being generated in one-pot and thus allow obtaining insights into multiple synthetic pathways at the same time. The reactivity of glyconitrile and the reactive species that generate from its initial chemical pathways allows the interaction with other nucleophilic or electrophilic precursors, for example of biologically active substances, that is typically used in combinatorial synthetic approaches and will lead to multiple derivatives that present substructures of such biologically active substances.

Additional chemical input in form of additional reagents may be fed to the glyconitrile CRN system based on possible target compounds. For instance, the formation of other amino acids, nucleotides and lipid precursor derivatives may be induced by adding glyceraldehyde, 2-aminooxazole, cyanoacetylene or mixtures thereof. Pyruvate and glyoxylate may also induce the formation of important metabolites from the human body, and, thus, are of therapeutic interest.

This new approach presents two main potential advantages: exploration of multiple synthetic pathways at the same time, and evaluation of positive and negative feedback effects between their activities, since the glyconitrile CRN allows studying the interactions between different species in the glyconitrile CRN and their respective interconversions, which is not available in combinatorial chemistry. In any case, available biological screening strategies are also applicable to glyconitrile CRNs. Hence, the method of implementation of the glyconitrile CRN may additionally comprise a step of (d) screening the biological activity of the glyconitrile CRN composition and use adequate means for performing this step.

Means for screening the biological activity may include liquid chromatography techniques at microscale because this will allow for separation of compounds by letting a mobile phase pass across them at high pressures. This kind of separation/fractionation of compounds from the glyconitrile CRN may be a necessary step prior to screening their biological activity. The screening of the biological activity will depend on the targeted molecules and the targeted biological and/or pharmacological candidate or candidates.

In a particular aspect herein, the variety of molecules with potential biological and/or pharmacological properties may include amino acids, nucleotides, lipid precursors, sugars metabolites, or their salts and derivatives of pharmaceutical drug leads if their precursors are added as additional chemical inputs to react with the glyconitrile CRN intermediates.

Most preferred aspects of this invention are as follows:
1. Method for implementing a glyconitrile chemical reaction network, the glyconitrile chemical reaction network being generated from a set of starting compounds comprising (a) glyconitrile; (b) a salt selected from the group consisting of alkali halides, alkaline earth metal halides, or mixtures thereof; and (c) a base selected from the group consisting of alkali hydroxides, alkaline earth metal hydroxides, ammonia, mono alkyl amines, dialkyl amines, trialkyl amines, alkali phosphates, alkaline earth metal phosphates, alkali carbonates, alkaline earth metal carbonates, or mixtures thereof.
2. The method of clause 1, wherein the glyconitrile chemical reaction network further comprises a solvent selected from the group consisting of water, or a combination of water and a water-miscible organic solvent.
3. The method of clause 2, wherein the solvent is water.
4. The method of clause 1, wherein the initial (a) glyconitrile concentration is from 250 mM to 1M, the initial (b) salt concentration is from 100 mM to 1 M, and the initial (c) base concentration is from 30 mM to 750 mM.
5. The method of clause 1, wherein the reaction temperature is chosen from 20-100 °C.
6. A glyconitrile chemical reaction network providing multiple compounds, wherein the glyconitrile chemical reaction network is generated from a set of starting compounds comprising (a) glyconitrile; (b) a salt; and (c) a base, wherein the initial (a) glyconitrile concentration is from 250 mM to 1 M, the initial (b) salt concentration is from 100 mM to 1 M, and the initial (c) base concentration is from 30 mM to 750 mM.
7. The glyconitrile chemical reaction network of clause 6, wherein (b) the salt is selected from the group consisting of alkali halides, alkaline earth metal halides, or mixtures thereof.
8. The glyconitrile chemical reaction network of clause 6, wherein (c) the base is selected from the group consisting of alkali hydroxides, alkaline earth metal hydroxides, ammonia, mono alkyl amines, dialkyl amines, trialkyl amines, alkali phosphates, alkaline earth metal phosphates, alkali carbonates, alkaline earth metal carbonates, or mixtures thereof.
9. The glyconitrile chemical reaction network of clause 6, wherein the glyconitrile chemical reaction network is provided as a solution comprising a solvent selected from the group consisting of water, or a combination of water and a water-miscible organic solvent.
10. The glyconitrile chemical reaction network of clause 6, wherein the temperature of the glyconitrile chemical reaction network is maintained at a temperature selected from the interval between 20 and 100 °C.
11. The glyconitrile chemical reaction network of clause 6, wherein the glyconitrile chemical reaction network is implemented in batch mode or in continuous mode.
12. A method for continuously modulating the glyconitrile chemical reaction network of clause 6 comprising the steps of providing at least one chemical input by means for a continuous reaction system having at least one inlet channel, analysing the composition of the glyconitrile chemical reaction network by means for analysis, and varying one or more chemical reaction conditions selected from the group of temperature, agitation speed, pH, reagent concentration, or reaction time.
13. A process for preparing amino acids, nucleobases, nucleotides, oligopeptides, and/or metabolite derivatives thereof using a glyconitrile chemical reaction network according to any of the preceding clauses, comprising the steps of generating and/or implementing a glyconitrile chemical reaction network, analysing the composition of the glyconitrile chemical reaction network after a defined set time, identifying a desired amino acid, nucleobase, nucleotide, oligopeptide, and/or metabolite derivative thereof, and modulating the reaction conditions in a way to predominately produce the desired amino acid, nucleobase, nucleotide, oligopeptide, and/or metabolite derivative thereof.
14. Use of the glyconitrile chemical reaction network of clause 6 or implemented according to clause 1 for a multi-compound screening of biologically active substances.
15. A device configured for implementing a glyconitrile chemical reaction network of clause 1 or clause 6 comprising (i) means for the preparation of the glyconitrile chemical reaction network, (ii) means for sampling the glyconitrile chemical reaction network composition one or more times at defined time points providing one or more sample datasets, (iii) means for analysing the one or more sample datasets, including means for data acquisition and data extraction, providing one or more analysis datasets, (iv) means for data processing of the obtained one or more analysis datasets, and (v) means for compounds identification.

### EXAMPLES

### General procedure for generation of the glyconitrile CRN

Different volumes of three different aqueous stock solutions containing glyconitrile (1M), NaOH (1M) and NaCl (2M) are mixed, so that their starting concentrations are within the following ranges: 0.32 - 0.62 M for glyconitrile; 50 - 110 mM for NaOH; and 0.1 - 0.4 M for NaCl. The reaction mixture is then heated at a temperature in the range between 40 - 70 °C, either in a test tube placed in an orbital shaker at 700 rpm (rpm = rounds per minute) or in a continuously stirred tank reactor (CSTR) where various inlet channels can be used to modify different chemical input variables.

The experiments in test tubes are considered to be in batch. This means that the input conditions cannot be recursively changed. Hence, the dependence of the glyconitrile CRN composition with the input conditions is established by analysing reactions with different concentrations of the three reagents, at different temperatures and at different points in time (all within the above-mentioned ranges). For the analysis of each sample, the test tube is removed from the orbital shaker and introduced in an ice bath to cool down the reaction. Thereafter, the pH is measured, and 0.3 mL of the composition are placed in a 1.5 mL Eppendorf tube. The solution is centrifuged at 14,000 rpm and 2 °C. A sample of 20 µL is then taken and deposited into an insert inside a 2 mL HPLC vial for the metabolomics analysis. In parallel, a 1 µL drop is deposited onto a Nanodrop instrument to record the UV-Vis spectrum (220-600 nm).

In the CSTR setup: it is possible to continuously change input conditions and to analyse the output composition after such a change, allowing to influence the glyconitrile CRN state in a recursive way. Experimentally, a CSTR with 3 input channels and one output was used. In a preferred configuration, inlet Channel 1 supplied a solution of 0.4 M NaCl and 2 M glyconitrile, inlet Channel 2 supplied a solution of 0.4 M NaCl and 1 M NaOH, and inlet Channel 3 supplied a solution of 0.4 M NaCl. The reactor had a volume of 470 µL, it was equipped with a magnet, and it was deposited on a temperature-controlled stirring plate.

In a typical experiment, starting from an initial reactor concentration of 0.4 M NaCl at 70 °C, the three inlet channels are activated at the same time with flow rates ranging from of 0 to 17 µL/min for the each of the channels. During the first minute, the output channel is pulled to waste. The outflow is then collected every 2 min in a 1.5 mL Eppendorf tube placed in an ice bath. The subsequent metabolomics analyses are carried out as described below.

### General procedure for metabolomics analysis of the glyconitrile CRN

In the conditions described above, hundreds of species with interconnected formation pathways are generated. In this context, the application of metabolomics analysis to a glyconitrile CRN is critical to identify as many species as possible and to highlight the highly intricate and non-linear dynamics of the network. These procedures are as follows.

For HPLC analyses of each input conditions applied in either the batch reactor or the CSTR, aliquots of 5 µL were injected into a HPLC model 1290 (Agilent Technologies, Germany), and compounds were separated using a Waters Acquity UPLC BEH Amide column (150 mm × 2.1 mm id; 1.7 µm particle size) equipped with a Waters Acquity UPLC BEH Amide VanGuard Pre-column (5 mm × 2.1 mm id, 1.7 µm). The column temperature is held at 45 °C and the flow rate is set at 0.4 mL/min. Milli-Q water is used as mobile phase (A) and 95:5 (v/v) acetonitrile:water is used as mobile phase (B), and 10 mM ammonium formate and 0.125% formic acid are used as a mobile phase modifiers. The gradient starts with 2 min at 100% (B), 100-70% (B) in 5.7 min, 70-40% (B) in 1.8 min, 40-30% (B) in 0.75 min, 30-100% (B) in 2.5 min, and 4 min at 100% (B) to come back to initial conditions. Compounds are eluted into a Q/TOF series 6540 from Agilent Technologies (Germany), equipped with an Agilent Jet Stream thermal orthogonal ESI source. The mass spectrometer is operated in ESI positive mode using the following parameters: capillary voltage, 3500 V; mass range, 50-1700 m/z; nebulizer pressure, 35 psig; drying gas flow rate, 11 L/min; dry gas temperature, 200 °C. The sheath gas flow is 11 L/min at 350 °C. MS/MS spectra are acquired employing the auto MS/MS mode using 4 precursors per cycle, dynamic exclusion after two spectra (released after 0.2 min), and collision energies of 20 and 40 V. Mass accuracy is corrected using ions at m/z 121.0509 (C5H4N4) and 922.0098 (C18H1806N3P3F24) simultaneously pumped into the ion source. Before and after the analysis of the samples, a mixture of standard compounds (CUDA, DL-alanine-d3, DL-glutamic acid-d3, d9-choline chloride, 15N2-L-arginine, L-methionine-d8 and Val-Tyr-Val) is injected as quality control sample and for retention time/compound identification purposes in the bioinformatics data processing stage. When possible, standard compounds are analysed under identical conditions to confirm their presence in the targeted samples.

The above HPLC analyses rendered massive data that needed to be extracted and processed. For data processing, raw data files were converted to ABF format using Reifycs Abf (Analysis Base File) Converter software (http://www.reifycs.com/AbfConverter/). Data extraction of ABF converted files was performed using MS-DIAL (v. 4.9.22) software (http://prime.psc.riken.jp/compms/msdial/main.html) for deconvolution, peak picking, alignment, and compound identification. The following parameters are used for data extraction: retention time, 0-17 min; mass range, 50-1700 Da; MS1 tolerance, 0.01 Da; MS2 tolerance, 0.025 Da; smoothing level, 3 scans; minimum peak width, 5 scans; minimum peak height, 1000; mass slice width, 0.1 Da; sigma window value for deconvolution, 0.1; retention time tolerance for alignment, 0.1 min; MS1 tolerance for alignment, 0.015 Da

For profile analysis, the alignment result (height data matrix) was exported in ".csv" format and the list of features was filtered by removing: i) unknown metabolites; ii) metabolites with a maximum height below 3000 units; iii) compounds appearing in control samples; iv) duplicated compounds; v) compounds identified as drugs; vi) compounds with not convincing MS/MS spectra; and vii) compounds with too complex chemical structure. Visualization of the results was performed by using Microsoft Office software. After these steps, the data is saved as ".txt" format to be processed by Perseus software (https://maxquant.net/perseus/) and to get an overview of the data through elaboration of heatmaps that relate compositional fingerprints with the reaction time or conditions.

A MS-based analytical platform was established to probe the network response to a broad range of experimental conditions, both in batch and in the CSTR. One approach to analyse, visualize and interpret the complexity of the data obtained from the glyconitrile CRN was to apply a non-supervised multivariate analysis, such as Principal Component Analysis, PCA. Also, heatmaps could be constructed that correlated the different observed species over time or with the matrix of employed conditions with the help of Perseus software.

For compound identification, in-house m/z and retention time (RT) libraries were used in addition to public MS/MS spectra databases in MSP format. The MSP file used for annotation was generated by combining MS/MS spectra from NIST MS/MS database, the LipidBLAST mass spectral library and MassBank of NorthAmerica. Complementary to the identification of compounds with authentic standards or by comparison with MS/MS spectral databases, other bioinformatic tools were applied to tentatively identify compounds not present in those databases. For instance, the MS/MS spectra of a list of potential compounds present in the glyconitrile CRN were predicted by using CFM-ID 4.0 web server and were subsequently compared to the experimental data. Moreover, raw data files were also converted to ".mzML" format using ProteoWizard software to make the files compatible with other bioinformatic tools such as SIRIUS, which performed an analysis and automatic interpretation of the MS/MS data to obtain molecular formulas, molecular formula ranking, fingerprint prediction, structure database search and compound class prediction. Finally, manual interpretation of the MS/MS spectra could be performed based on the fragmentation rules extracted from confirmed compounds.

### Example 1: Monitoring of the reaction dynamics over time in batch experiments.

The following stock solutions are prepared to be used in the different experiments:
Glyconitrile 1 M (10 mL): 9.24 mL of Milli-Q water and 0.76 mL of a 70% glyconitrile solution (Sigma-Aldrich) were added to a vial. The mixture was vortexed for 30 seconds and then used as required.
NaCl 2 M (10 mL): 1.17 g of NaCl were weighed into a vial. 10 mL of Milli-Q water were then added, and the mixture was shaken until no precipitate was observed.
NaOH 1 M (10 mL): 400 mg of NaOH were weighed in a vial. 10 mL of Milli-Q water were then added, and the mixture was shaken until no precipitate is observed.

The experiment was carried out at 70 °C with the initial input concentrations of 520 mM glyconitrile, 110 mM NaOH and 400 mM NaCl.

In a vial placed in an ice bath, 2.55 mL of Milli-Q water, 3 mL of 2 M NaCl solution, 7.8 mL of 1 M glyconitrile solution and 1.65 mL of 1 M NaOH solution were added. The mixture was divided into 15 test tubes (0.8 mL, 85 X 15 mm), thus having a sample for each time to be evaluated. The test tubes were placed in an orbital shaker at 700 rpm and 70 °C. The total reaction time was 258 min, analysing a sample every 18 minutes. The total number of time points to be evaluated was 15, including a zero time.

For the analysis of each sample, the respective test tube was removed from the orbital shaker and introduced in an ice bath to cool down the reaction. Thereafter, the pH was measured, and a volume of 0.3 mL was placed in a 1.5 mL Eppendorf tube. The solution was centrifuged at 14,000 rpm and 2 °C. 20 µL were then taken and deposited into an insert inside a 2 mL HPLC vial for the metabolomic analysis. In parallel, a 1 µL nanodrop was deposited to perform the UV-Vis measurement (220-600 nm).

For HPLC analyses, aliquots of 5 µL were injected into a HPLC model 1290 (Agilent Technologies, Germany), and compounds were separated using a Waters Acquity UPLC BEH Amide column (150 mm × 2.1 mm id; 1.7 µm particle size) equipped with a Waters Acquity UPLC BEH Amide VanGuard Pre-column (5 mm × 2.1 mm internal diameter (id), 1.7 µm particle size). The column temperature was held at 45 °C and the flow rate was set at 0.4 mL/min. Milli-Q water was used as mobile phase (A) and 95:5 (v/v) acetonitrile water was used as mobile phase (B), and 10 mM ammonium formate and 0.125% formic acid were used as a mobile phase modifiers. The gradient started with 2 min at 100% (B), 100-70% (B) in 5.7 min, 70-40% (B) in 1.8 min, 40-30% (B) in 0.75 min, 30-100% (B) in 2.5 min, and 4 min at 100% (B) to come back to initial conditions. Compounds were eluted into a Q/TOF series 6540 from Agilent Technologies (Germany), equipped with an Agilent Jet Stream thermal orthogonal ESI source.

The mass spectrometer was operated in ESI positive mode using the following parameters: capillary voltage, 3500 V; mass range, 50-1700 m/z; nebulizer pressure, 35 psig (equivalent to 241317 pascal); drying gas flow rate, 11 L/min; dry gas temperature, 200 °C. The sheath gas flow was 11 L/min at 350 °C. MS/MS spectra were acquired employing the auto MS/MS mode using 4 precursors per cycle, dynamic exclusion after two spectra (released after 0.2 min), and collision energies of 20 and 40 V. Mass accuracy was corrected using ions at m/z 121.0509 (C₅H₄N₄) and 922.0098 (C₁₈H₁₈O₆N₃P₃F₂₄) simultaneously pumped into the ion source. Before and after the analysis of the samples, a mixture of standard compounds (12-[[cyclohexylamino)carbonyl]amino]-dodecanoic acid (CUDA), DL-alanine-d₃, DL-glutamic acid-d₃, d₉-choline chloride, 15N₂-L-arginine, L-methionine-d₈ and Val-Tyr-Val) was injected as quality control sample and for retention time/compound identification purposes in the bioinformatics data processing stage. When possible, standard compounds were analysed under identical conditions to confirm their presence in the targeted samples.

For data processing, raw data files were converted to ABF format using Reifycs Abf (Analysis Base File) Converter software (http://www.reifycs.com/AbfConverter/). Data extraction of ABF converted files was performed using MS-DIAL (v. 4.9.22) software (http://prime.psc.riken.jp/compms/msdial/main.html) for deconvolution, peak picking, alignment, and compound identification. The following parameters were used for data extraction: retention time, 0-17 min; mass range, 50-1700 Da; MS1 tolerance, 0.01 Da; MS2 tolerance, 0.025 Da; smoothing level, 3 scans; minimum peak width, 5 scans; minimum peak height, 1000; mass slice width, 0.1 Da; sigma window value for deconvolution, 0.1; retention time tolerance for alignment, 0.1 min; MS1 tolerance for alignment, 0.015 Da.

For profile analysis, the alignment result (height data matrix) was exported in ".csv" format and the list of features was filtered by removing: i) unknown metabolites; ii) metabolites with a maximum height below 3000 units; iii) compounds appearing in control samples; iv) duplicated compounds; v) compounds identified as drugs; vi) compounds with not convincing MS/MS spectra; and vii) compounds with too complex chemical structure. Visualization of the results was performed by using Microsoft Office software. After these steps, the data was saved as ".txt" format to be processed by Perseus software (https://maxquant.net/perseus/) and to get an overview of the data through elaboration of heatmaps that relate compositional fingerprints with the reaction time or conditions.

For compound identification, in-house m/z and retention time (RT) libraries were used in addition to public MS/MS spectra databases in MSP format. The following parameters were selected: accurate mass tolerance (MS1 and MS2) for MSP library, 0.01 Da and 0.025 Da; identification score cut off for MSP library, 80%; retention time tolerance for RT-m/z library, 0.1 min; accurate mass tolerance for RT-m/z library, 0.01 Da; identification score cut off for RT-m/z library, 85%. CUDA, DL-alanine-d₃, DL-glutamic acid-d₃, d₉-choline chloride, 15N₂-L-arginine, L-methionine-d₈ and Val-Tyr-Val standard compounds were used for retention time correction and for compound identification using the RT-m/z library. The MSP file used for annotation was generated by combining MS/MS spectra from NIST MS/MS database, the LipidBLAST mass spectral library and MassBank of NorthAmerica (MoNA available at https://mona.fiehnlab.ucdavis.edu/).

Complementary to the identification of compounds with authentic standards or by comparison with MS/MS spectral databases, other bioinformatic tools were applied to tentatively identify other compounds not present in those databases. For instance, the MS/MS spectra of a list of potential compounds present in the glyconitrile CRN were predicted by using CFM-ID 4.0 web server (https://cfmid.wishartlab.com/) and were subsequently compared to the experimental data. Moreover, raw data files were also converted to ".mzML" format using ProteoWizard software (https://proteowizard.sourceforge.io/) to make the files compatible with other bioinformatic tools such as SIRIUS (https://bio.informatik.uni-jena.de/software/sirius/), which performs an analysis and automatic interpretation of the MS/MS data to obtain molecular formulas, molecular formula ranking, fingerprint prediction, structure database search and compound class prediction. Finally, a manual interpretation of the MS/MS spectra was performed based on the fragmentation rules extracted from confirmed compounds, such as N-(2-acetamido)iminodiacetic acid.

### Example 2: Monitoring of the reaction dynamics over time in flow (CSTR).

The following stock solutions are prepared to be used in the different experiments:
NaCl 0.472 M (10 mL): 276 mg of NaCl were weighed into a vial. 10 mL of Milli-Q water were then added, and the mixture was shaken until no precipitate was observed.
NaCl 0.4 M (20 mL): 468 mg of NaCl were weighed into a vial. 20 mL of Milli-Q water were then added, and the mixture was shaken until no precipitate was observed.
NaOH 1 M (10 mL): 200 mg of NaOH were weighed in a vial. 10 mL of a solution of NaCl (0.4 M) were then added and the mixture was shaken until no precipitate was observed.
Glyconitrile 2 M (10 mL): 8.48 mL of a solution of NaCl (0.472 M) and 1.52 mL of a 70% glyconitrile solution (Sigma-Aldrich) were added to a vial. The mixture was vortexed for 30 s.

The experiment was carried out at 70 °C with a residence time of 20 min, and the initial input concentrations of 520 mM glyconitrile, 110 mM NaOH and 400 mM NaCl.

A CSTR with 3 input channels and one output was used. Inlet Channel 1 was fed with a solution of 0.4 M NaCl and 2 M glyconitrile; inlet Channel 2 was fed with a solution of 0.4 M NaCl and 1 M NaOH; and inlet Channel 3 only was supplied with a solution of 0.4 M NaCl. The reactor had a volume of 470 µL, it was equipped with a magnet, and it was deposited on a temperature-controlled stirring plate.

Starting from an initial reactor concentration of 0.4 M NaCl at 70 °C, the three inlet channels were then activated at the same time with flow rates of 14 µL/min for Channel 1, 2.5 µL/min for Channel 2 and 5.8 µL/min for Channel 3. The first minute of the output channel was pulled to waste. The outflow was then collected for 2 min in a 1.5 mL Eppendorf tube placed in an ice bath. The sample was centrifuged at 14,000 rpm and 2 °C. From the supernatant, 20 µL were taken and deposited into an insert inside a 2 mL HPLC vial for the metabolomics analysis. Thereafter, the CSTR system was left to elapse during 16 min where the output current went to waste. After this time, the outflow was collected again for 2 min and the same steps described above were repeated. This process was carried out until a time of 75 min was reached.

The subsequent HPLC-MS analyses, data processing and compound identification steps were performed in the same manner as described for Example 1.

### Example 3: Evaluation of a matrix of different input conditions in batch.

The same stock solutions described in Example 1 were used in this example.

The experiment was performed with glyconitrile at three different concentrations (0.52, 0.42 and 0.32 M), NaOH at three different concentrations (0.11, 0.075 and 0.05 M) and three different temperatures (40, 55 and 70 °C), whereas NaCl concentration was always constant in each case at 0.4 M and reaction time was always 30 minutes.

The general experimentation procedure is shown based on the following exemplary conditions: a concentration of 0.52 M glyconitrile and 0.11 M NaOH at 70 °C.

In a vial placed in an ice bath, 0.24 mL of Milli-Q water, 0.4 mL of 2 M NaCl solution, 1.04 mL of 1 M glyconitrile solution and 0.22 mL of 1 M NaOH solution were added. From this solution 0.8 mL were taken and added in a test tube (0.8 mL, 85 X 15 mm). The test tube was placed in an orbital shaker at 700 rpm and 70 °C.

After 30 min, the test tube was removed from the orbital shaker and introduced in an ice bath to cool down the reaction. Thereafter, the pH was measured, and a volume of 0.3 mL was placed in a 1.5 mL Eppendorf tube. The solution was centrifuged at 14,000 rpm and 2 °C. From here, 20 µL were taken and deposited into an insert inside a 2 mL HPLC vial for the metabolomics analysis.

Blank experiments for comparison: Instead of the glyconitrile solution (1 M), the same volume of water was used to prepare blanks for the different experiment conditions. The rest of the procedures for other samples preparation and analysis are the same as described above. In these blank samples, no additional signals (other than those coming from the mobile phases and the analytical instrument) were obtained after the HPLC-MS analysis, and the chromatograms were identical independently of the temperature or NaOH concentration used. These results indicate the necessity of including glyconitrile solution to generate the glyconitrile CRN.

The subsequent HPLC-MS analyses, data processing and compound identification steps were performed in the same manner as described for Example 1.

### Example 4: Response surface experiment for predominant production of target molecules

The same stock solutions described in Example 1 were used in this example.

To explore the potential predominant production of certain features or molecules of interest by modulating the reaction conditions, a response surface methodology (RSM) was performed based on two different central composite designs (CCD). In the first one, the reaction time was set to 30 minutes and three factors with three levels (3³) were employed, namely glyconitrile concentration (0.32 M, 0.42 M, 0.52 M), NaOH concentration (0.05 M, 0.075 M, 0.11 M) and temperature (T) (40°C, 55°C, 70°C). In the second one, the reaction time was set to 30 min, the glyconitrile concentration was fixed at 0.52 M, and two factors with five levels (5²) were employed, namely NaOH concentration (0.05 M, 0.07 M, 0.09, 0.11 M, 0.13 M) and temperature (40°C, 47.5°C, 55°C, 62.5°C, 70°C).

Different response variables may be studied to demonstrate the feasibility of this approach. For instance, for 3-methyladenine, the RSM based on the 3³ CCD forecasted that the maximization of its production occurred (desirability = 0.791) when 0.52 M Gly, 0.11 M NaOH and 70°C were used; and the RSM based on the 5² CCD forecasted that maximization happened (desirability = 0.995) when 0.13 M NaOH and 70°C were used. Similarly, for 2-(hydroxymethyl)-1,4-dihydroimidazol-5-one, the RSM based on the 3³ CCD forecasted that the maximization of its production occurred (desirability = 0.983) when 0.52 M Gly, 0.05 M NaOH and 40°C were used; and the RSM based on the 5² CCD forecasted that maximization happened (desirability = 0.904) when 0.05 M NaOH and 40.5°C were used.

The subsequent HPLC-MS analyses, data processing and compound identification steps were performed in the same manner as described for Example 1.

## Claims

1. Method for implementing a glyconitrile chemical reaction network, the glyconitrile chemical reaction network being generated from a set of starting compounds comprising (a) glyconitrile; (b) a salt selected from the group consisting of alkali halides, alkaline earth metal halides, or mixtures thereof; and (c) a base selected from the group consisting of alkali hydroxides, alkaline earth metal hydroxides, ammonia, mono alkyl amines, dialkyl amines, trialkyl amines, alkali phosphates, alkaline earth metal phosphates, alkali carbonates, alkaline earth metal carbonates, or mixtures thereof.

2. The method of claim 1, wherein the glyconitrile chemical reaction network further comprises a solvent selected from the group consisting of water, or a combination of water and a water-miscible organic solvent.

3. The method of claim 2, wherein the solvent is water.

4. The method of claim 1, wherein the initial (a) glyconitrile concentration is from 250 mM to 1M, the initial (b) salt concentration is from 100 mM to 1 M, and the initial (c) base concentration is from 30 mM to 750 mM.

5. The method of claim 1, wherein the reaction temperature is chosen from 20-100 °C.

6. A glyconitrile chemical reaction network providing multiple compounds, wherein the glyconitrile chemical reaction network is generated from a set of starting compounds comprising (a) glyconitrile; (b) a salt; and (c) a base, wherein the initial (a) glyconitrile concentration is from 250 mM to 1 M, the initial (b) salt concentration is from 100 mM to 1 M, and the initial (c) base concentration is from 30 mM to 750 mM.

7. The glyconitrile chemical reaction network of claim 6, wherein (b) the salt is selected from the group consisting of alkali halides, alkaline earth metal halides, or mixtures thereof.

8. The glyconitrile chemical reaction network of claim 6, wherein (c) the base is selected from the group consisting of alkali hydroxides, alkaline earth metal hydroxides, ammonia, mono alkyl amines, dialkyl amines, trialkyl amines, alkali phosphates, alkaline earth metal phosphates, alkali carbonates, alkaline earth metal carbonates, or mixtures thereof.

9. The glyconitrile chemical reaction network of claim 6, wherein the glyconitrile chemical reaction network is provided as a solution comprising a solvent selected from the group consisting of water, or a combination of water and a water-miscible organic solvent.

10. The glyconitrile chemical reaction network of claim 6, wherein the temperature of the glyconitrile chemical reaction network is maintained at a temperature selected from the interval between 20 and 100 °C.

11. The glyconitrile chemical reaction network of claim 6, wherein the glyconitrile chemical reaction network is implemented in batch mode or in continuous mode.

12. A method for continuously modulating the glyconitrile chemical reaction network of claim 6 comprising the steps of providing at least one chemical input by means for a continuous reaction system having at least one inlet channel, analysing the composition of the glyconitrile chemical reaction network by means for analysis, and varying one or more chemical reaction conditions selected from the group of temperature, agitation speed, pH, reagent concentration, or reaction time.

13. A process for preparing amino acids, nucleobases, nucleotides, oligopeptides, and/or metabolite derivatives thereof using a glyconitrile chemical reaction network according to any of the preceding claims, comprising the steps of generating and/or implementing a glyconitrile chemical reaction network, analysing the composition of the glyconitrile chemical reaction network after a defined set time, identifying a desired amino acid, nucleobase, nucleotide, oligopeptide, and/or metabolite derivative thereof, and modulating the reaction conditions in a way to predominately produce the desired amino acid, nucleobase, nucleotide, oligopeptide, and/or metabolite derivative thereof.

14. Use of the glyconitrile chemical reaction network of claim 6 or implemented according to claim 1 for a multi-compound screening of biologically active substances.

15. A device configured for implementing a glyconitrile chemical reaction network of claim 1 or claim 6 comprising (i) means for the preparation of the glyconitrile chemical reaction network, (ii) means for sampling the glyconitrile chemical reaction network composition one or more times at defined time points providing one or more sample datasets, (iii) means for analysing the one or more sample datasets, including means for data acquisition and data extraction, providing one or more analysis datasets, (iv) means for data processing of the obtained one or more analysis datasets, and (v) means for compounds identification.
